# EUROPEAN PATENT APPLICATION

(11) **EP 3 715 895 A1**
(43) Date of publication of application: **30.09.2020**
(21) Application number: 19165617.2
(22) Date of filing: 27.03.2019
(51) Int. Cl.: G01R 33/563, G01R 33/483, G01R 33/565, A61B 5/00

(54) **SLICE ALIGNMENT FOR SHORT AXIS CARDIAC MR CINE SLICE STACKS**

(71) Applicant: Koninklijke Philips N.V., 5656 AG Eindhoven (NL)
(72) Inventor: PETERS, Jochen, 5656 AE Eindhoven (NL); WEESE, Rolf Jürgen, 5656 AE Eindhoven (NL); WISSEL, Tobias, 5656 AE Eindhoven (NL); WEBER, Frank Michael, 5656 AE Eindhoven (NL)
(74) Representative: de Haan, Poul Erik

(57) **Abstract**

Slice alignment approaches are described for short axis cardiac magnetic resonance cine slice stacks, which do not require additional scans, such as long axis scans or full 3D scans, and which are able to deal with cardiac structures having complex shapes. Both approaches do not need contours to follow a quadratic curvature function, and are well suitable for the purpose of obtaining a segmentation of a cardiac structure using a deformable surface model. Namely, such a deformable surface model is unable, but also not desired, to fully adapt to the 'zig-zag'-shaped pattern in the boundary of the cardiac structure due to the slice misalignment. Having removed or reduced the misalignment between image slices, such a deformable surface model may better adapt to the cardiac structure in the image data and thereby provide a better segmentation of the cardiac structure.

## Description

### FIELD OF THE INVENTION

The invention relates to systems and computer-implemented methods for slice alignment of short axis cardiac magnetic resonance (MR) cine slice stacks. The invention further relates to a machine trained model for slice alignment of short axis cardiac MR cine slice stacks, and to a computer-readable medium comprising instructions to perform one of the computer-implemented methods.

### BACKGROUND OF THE INVENTION

Cardiac MR images are typically acquired in a series of cine acquisitions of short axis slices, and time delays between the multi-breath-hold single slice acquisitions lead to a misalignment which complicates 3D interpretation. 3D interpretation is useful for many advanced medical analyses (e.g. accurate definition of apex and valves, wall thickness measurements, improved volume calculation as compared to the Simpson method, etc.)

Aligning the slice stack is a non-trivial task. For example, simply stacking the left ventricular blood-pool regions (roughly circular shaped in short axis slices) on a common straight axis does not correspond to the real anatomy which shows a curved blood-pool centreline. The same holds for the right ventricle contours that follow a non-trivial curved shape. Hence, a simple registration maximizing the similarity of registered slices may not yield an anatomically correct alignment. In addition, since slices are typically thick (8-10mm), the content of successive slices may differ considerably, complicating slice-to-slice registration.

US 2017109881 A1 describes aligning short-axis images of the heart chamber by performing contour alignment to reduce misalignment between the short-axis images. The contours are assumed to follow a quadratic curvature function of which the parameters are estimated by minimizing a mean squared error. The contours are registered using an affine transformation with linear interpolation, according to the estimated center values to obtain an aligned stack of contours.

Disadvantageously, US 2017109881 A1 assumes the contours to follow a quadratic curvature function, which is not always the case. For example, the right ventricle contours typically follow a more complex curved shape.

### SUMMARY OF THE INVENTION

It may be desirable to obtain a system and computer-implemented method for slice alignment of short axis cardiac magnetic resonance (MR) cine slice stacks which is better able to deal with cardiac structures of varying shapes.

In accordance with a first aspect of the invention, a system is provided for slice alignment of short axis cardiac magnetic resonance cine slice stacks. The system comprises:
- an input interface for accessing image data of an input set of image slices acquired using a short axis cardiac magnetic resonance cine protocol;
- a processor subsystem configured to:
   - access trained model data defining a machine trained model, wherein the machine trained model is trained on training data comprising image data of a training set of image slices acquired using a short axis cardiac magnetic resonance cine protocol, wherein one or more adjacent image slices are mutually misaligned, and wherein the training data further comprises shift values for reducing said mutual misalignment by shifting one or more of the image slices;
   - apply the machine trained model to sets of adjacent image slices of the input set of image slices, thereby obtaining at least one shift value for at least one of the image slices of the sets of adjacent image slices; and
   - shift said image slice based on the shift value.

In accordance with a further aspect of the invention, a computer-implemented method is provided for slice alignment of short axis cardiac magnetic resonance cine slice stacks. The method comprises:
- accessing image data of an input set of image slices acquired using a short axis cardiac magnetic resonance cine protocol;
- accessing trained model data defining a machine trained model, wherein the machine trained model is trained on training data comprising image data of a training set of image slices acquired using a short axis cardiac magnetic resonance cine protocol, wherein one or more adjacent image slices are mutually misaligned, and wherein the training data further comprises shift values for reducing said mutual misalignment by shifting one or more of the image slices;
- applying the machine trained model to sets of adjacent image slices of the input set of image slices, thereby obtaining at least one shift value for at least one of the image slices of the sets of adjacent image slices; and
- shifting said image slice based on the shift value.

In accordance with a further aspect of the invention, a computer-readable medium is provided comprising transitory or non-transitory data defining a machine trained model, wherein the machine trained model is configured and trained to be applied to a set of adjacent image slices of a set of image slices acquired using a short axis cardiac magnetic resonance cine protocol, wherein the machine trained model is trained to output a shift value if the set of adjacent image slices is mutually misaligned for reducing mutual misalignment.

In accordance with a further aspect of the invention, a system is provided for slice alignment of short axis cardiac magnetic resonance cine slice stacks. The system comprises:
- an input interface for accessing image data representing a set of image slices acquired using a short axis cardiac magnetic resonance cine protocol;
- a processor subsystem configured to:
   - access surface model data defining a deformable surface model for segmenting a cardiac structure in short axis cardiac MR cine slice stacks, wherein deformability of the surface model is constrained by shape regularization;
   - adapt the surface model to the cardiac structure by detecting boundary points of the cardiac structure in the image data and deforming the surface model towards the boundary points to obtain an adapted surface model which is adapted in shape to the cardiac structure in the image data; and
   - shift at least one image slice relative to other image slices so that the boundary points in the image slice obtain an improved match with a cross-sectional representation of the surface model in the respective image slice.

In accordance with a further aspect of the invention, a computer-implemented method is provided for slice alignment of short axis cardiac magnetic resonance cine slice stacks. The method comprises:
- accessing image data representing a set of image slices acquired using a short axis cardiac magnetic resonance cine protocol;
- accessing surface model data defining a deformable surface model for segmenting a cardiac structure in short axis cardiac MR cine slice stacks, wherein deformability of the surface model is constrained by shape regularization;
- adapting the surface model to the cardiac structure by detecting boundary points of the cardiac structure in the image data and deforming the surface model towards the boundary points to obtain an adapted surface model which is adapted in shape to the cardiac structure in the image data; and
- shifting at least one image slice relative to other image slices so that the boundary points in the image slice obtain an improved match with a cross-sectional representation of the surface model in the respective image slice.

In accordance with a further aspect of the invention, a computer-readable medium is provided comprising transitory or non-transitory data representing a computer program, the computer program comprising instructions for causing a processor system to perform one or both of the above methods.

The above measures provide solutions to slice alignment of short axis cardiac magnetic resonance cine slice stacks which do not require additional scans, such as long axis scans or full 3D scans, and which are better able to deal with cardiac structures having complex shapes. Compared to US 2017109881 A1, both approaches do not need contours to follow a quadratic curvature function.

In some embodiments, both approaches may be used cooperatively in that a slice stack of the slice alignment which is aligned using the deformable surface model may then be purposefully misaligned and, together with the shift values representing the misalignment, be used to train the machine trained model.

In general, both approaches operate on image data of an input set of image slices acquired using a short axis cardiac magnetic resonance cine protocol. Such an input set of image slices is elsewhere also referred to as an image stack or slice stack. As output, an aligned set of image slices may be obtained, or at least a better aligned set of image slices in which at least one of the image slices is shifted.

With further reference to the slice alignment using a machine trained model, the following is noted. The machine trained model may be configured and trained to be applied to sets of adjacent image slices, such as a pair or 3 or 4 adjacent image slices, and to provide as output at least one shift value for at least one of the image slices which may be used to improve the mutual alignment of the set of adjacent image slices if the respective image slice is shifted based on the shift value. Such a shift value may be expressed in any suitable format, such as a 2D vector defining a horizontal and vertical displacement, and may be expressed in various quantities, such as pixels or coordinates in a coordinate system associated with the image stack. In some embodiments, the shift value may only indirectly define a shift, in that it may express the misalignment, e.g., as a vector, with the shift being needed to correct the misalignment being equal to minus said vector. As such, the term 'shift value' is to be interpreted as a value which is indicative of the shift to be applied, and the shifting of an image slice 'based on' the shift value may comprise a function being applied to the shift value, such as inverting a sign of the shift value.

The machine trained model may be trained to operate on the image data of the adjacent image slices itself. For example, the machine trained model may be configured and trained to use image intensities values as input which may be sampled from each of the adjacent slices on a pre-defined grid (e.g., for each slice using 256 x 256 sampling points at known intervals, such as 1mm apart). In some embodiments, the machine trained model may directly use such sampled image intensity values as input, while in other embodiments, the machine trained model may use a difference between the image intensity values across the image slices as input. Here, 'across' the image slices may refer to the difference being calculated between the samples located at corresponding position in each image slice.

Effectively, the above approach addresses slice shift estimation as a regression task which is then solved by a machine trained model, for example one which is based on and trained using deep learning (neural network) techniques.

With further reference to the slice alignment using a deformable surface model, the following is noted. Deformable surface models are known per se, and may define a deformable surface for segmenting a structure, but which deformable surface may be constrained in terms of shape deformability by shape regularization. An example of such a deformable surface model is described in the publication titled *"*Shape-constrained deformable models and applications in medical imaging" in 'Shape Analysis in Medical Image Analysis', pp. 151-184 and authored by (a subset of) the inventors. The inventors have considered to use such a deformable surface model to guide the slice alignment. Namely, a surface model for a cardiac structure may be adapted to the cardiac structure in a manner as known per se, namely by detecting boundary points of the cardiac structure in the image data and deforming the surface model towards the boundary points to obtain an adapted surface model which is adapted in shape to the cardiac structure in the image data. However, due the shape regularization, the deformable surface model is likely to adapt to the general shape of the cardiac structure in the image stack, but is unlikely or less likely to adapt to the typically 'zig-zag'-shaped pattern in the boundary of the cardiac structure due to the misalignment between the image slices. Having adapted the surface model to the image stack, the adapted surface model may thus act as a reference for correcting the slice misalignment, in that individual slices may be shifted so that the detected boundary points of the cardiac structure in a slice better match the cross-section of the surface model in the respective slice.

Both approaches are well suitable for the purpose of obtaining a segmentation of a cardiac structure using a deformable surface model. Namely, such a deformable surface model is unable, but also not desired, to adapt to the 'zig-zag'-shaped pattern in the boundary of the cardiac structure due to the slice misalignment. Having removed or reduced the misalignment between image slices, such a deformable surface model may better adapt to the cardiac structure in the image data and thereby provide a better segmentation of the cardiac structure. Accordingly, the above measures may in both cases be followed by an application and adaptation of a deformable surface model to the image stack, or if such a deformable surface model has already been applied and adapted to the image data, by another iteration of adapting the deformable surface model to the image data.

The following optional aspects refer to the system and computer-implement method for slice alignment on the basis of a machine trained model. However, these optional aspects may, where applicable, denote corresponding modifications of the slice alignment on the basis of a deformable surface model.

Optionally, the processor subsystem is configured to:
- apply the machine trained model to the sets of adjacent image slices of the input set of image slices to obtain a series of shift values;
- remove an offset or linear trend from the series of shift values;
- shift respective image slices of the sets of adjacent image slices based on respective shift values of the series of shift values.

The inventors have recognized that the estimation of slice shifts may sometimes be imperfect, and that the slice alignment may, in particular when applied iteratively, e.g., from one slice to the next, cause slow translational drift and tilting (e.g., skewing) of the overall slice stack. This may be avoided or reduced by detecting and subsequently removing an offset or a linear trend from the series of shift values before applying the shift values to generate the aligned slice stack.

Optionally, the machine trained model is configured and trained to use as further input positional information which is indicative of a position of respective sets of adjacent image slices relative to a cardiac structure which is shown in the input set of image slices. A slice may show a particular part of the cardiac structure and may therefore have an anatomical position, e.g., a position relative to the cardiac structure. For example, a given slice A may have an anatomical position defined as x% of a range in which 0% is most apical or even below the apex and 100% is most basal or above the ventricles. Such anatomical positional information may be used as further input to the machine trained model, e.g., during training and subsequent use, to better address variation in the input data population.

Optionally, the machine trained model is configured and trained to use as further input angular information which is indicative of an orientation of a cardiac structure which is shown in the input set of image slices, relative to a coordinate system associated with the input set of image slices. Similar to using the anatomical position of the cardiac structure, the orientation of the cardiac structure in the slice stack may be used as further input to the machine trained model, e.g., during training and subsequent use, to better address variation in the input data population.

Optionally, the processor subsystem is configured to obtain at least one of the positional information and the angular information by segmenting the cardiac structure in the input set of image slices, for example by applying a deformable surface model to the image data. A deformable surface model may, when at least coarsely adapted to the image data, indicate the position and orientation of the cardiac structure relative to the slice stack. Such a deformable surface model may in some embodiments be present for also for the overall purpose of obtaining a segmentation of the cardiac structure, and may already be adapted (at least coarsely) to the image data before or during the slice alignment.

Optionally, the processor subsystem is configured to mask a part of the image data of the input set of image slices which does not belong to the cardiac structure before applying the machine trained model to the sets of adjacent image slices of the input set of image slices. The slice stack may show anatomical structures other than the cardiac structure, such as a ribcage surrounding the moving heart. Such other anatomical structures may undergo different transformations and may thereby disturb the slice alignment. By masking out such parts, e.g., based on a rough pre-segmentation or localization of the cardiac structure, the machine trained model may, during training and subsequent use, be steered to more focus the slice alignment on the cardiac structure.

Optionally, the input set of image slices is a first set of image slices, wherein the input interface is configured for accessing image data of a second set of image slices acquired during a different cardiac phase than the first set of image slices, and wherein the machine trained model is configured and trained to use spatially corresponding samples of the first set of image slices and the second set of image slices as joint input. Slice stacks may be acquired and available for different heart phases, e.g., for end-diastole (ED) and end-systole (ES) and possibly other heart phases. Such slice stacks may be sampled using a same sampling grid, e.g., using a same acquisition geometry of the MR acquisition apparatus. The intensity values from both slice stacks may be presented to the machine trained model, e.g., during training and subsequent use, as a joint input, e.g., as a vector of two or more intensity values. Thereby, one sample input of the machine trained model may comprise intensity values from different heart phases. Here, small or no differences between the intensity values may represent regions of regions of little motion (or homogeneous areas) whereas regions with large differences in intensity value may indicate motion. Such differences may be most prominent in the heart wall and may steer the machine trained model to more focus the slice alignment on the cardiac structure. Furthermore, by using the information of two or more heart phases as input to the machine trained model, during training and subsequent use, the estimation of the misalignment by the machine trained model may be more robust and less sensitive to noise.

Optionally, the input set of image slices comprises a cardiac structure, and wherein the processor subsystem is configured to resample the image data of the input set of image slices to show the cardiac structure at an anatomical standard position and/or orientation. Such resampling may bring the cardiac structure into an anatomical standard position and/or orientation in the slice stack. Such resampling may be performed explicitly, e.g., yielding a resampled slice stack, or implicitly, e.g., yielding a new sampling grid which may be used to access the image data of the slice stack when estimating and performing the slice alignment. In the latter case, the new sampling grid may effectively be used as a coordinate transformation which causes the machine trained model to access the image data of the cardiac structure in a standardized manner in terms of anatomical position and/or orientation.

The following optional aspects refer to the system and computer-implement method for slice alignment on the basis of a deformable surface model. However, these optional aspects may, where applicable, denote corresponding modifications of the slice alignment on the basis of a machine trained model.

Optionally, the processor subsystem is configured to deform the surface model towards the boundary points of the cardiac structure based on a cost function penalizing a distance of the surface model to the boundary points, and to shift the at least one image slice relative to the other image slices so that the match is improved according to the cost function. Such types of cost functions are known per se, and may also be known as an 'external energy term' or 'data fit term'.

Optionally, the processor subsystem is configured for iterative slice alignment by repeating said adapting of the surface model and said shifting of the at least one image slice at least twice. The adaptation of the surface model and the slice alignment based on the adapted surface model may be performed iteratively, in that both steps may be repeated at least twice. With a gradual decrease in slice misalignment, the surface model may be gradually adapted to better fit the image data.

It will be appreciated by those skilled in the art that two or more of the above-mentioned embodiments, implementations, and/or optional aspects of the invention may be combined in any way deemed useful.

Modifications and variations of a system, computer-implemented method and/or any computer program product, which correspond to the described modifications and variations of another one of said entities, can be carried out by a person skilled in the art on the basis of the present description.

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 shows a system for slice alignment of short axis cardiac magnetic resonance cine slice stacks, which in one embodiment may use a machine trained model and, in another embodiment, a deformable surface model;
Fig. 2 shows a system for training a machine trainable model for slice alignment of short axis cardiac magnetic resonance cine slice stacks;
Fig. 3 shows a cross-section of a misaligned slice stack, showing boundary points of a cardiac structure and an applied deformable surface model;
Fig. 4 shows the slice stack of Fig. 3 after shifting several slices to bring the boundary points into alignment with the applied deformable surface model;
Fig. 5 shows a set of adjacent slices and sampling grids indicating which intensity values are used as input for the machine trained model;
Fig. 6 shows adjacent slices of two slice stacks acquired during different cardiac phases, in which sampling grids are defined in both slice stacks;
Fig. 7 shows an image slice of a cardiac structure and a cross-section of the applied deformable surface model which is shown to be misaligned with the boundary of the cardiac structure due to a misalignment between image slices;
Fig. 8 shows the slice stack of Fig. 7 after shifting the image slice;
Fig. 9 shows a computer-implemented method for slice alignment using a machine trained model;
Fig. 10 shows a computer-implemented method for slice alignment using a deformable surface model; and
Fig. 11 shows a computer-readable medium comprising data.

It should be noted that the figures are purely diagrammatic and not drawn to scale. In the figures, elements which correspond to elements already described may have the same reference numerals.

### List of reference numbers

The following list of reference numbers is provided for facilitating the interpretation of the drawings and shall not be construed as limiting the claims.
- 020, 022: data storage
- 030, 032: short axis cardiac MR cine slice stack
- 040: shift values
- 050: trained model data
- 060: surface model data

- 100: system for slice alignment
- 120: input interface
- 122, 124: data communication
- 140: processor subsystem
- 160: communication interface

- 200: system for training model for slice alignment
- 220: input interface
- 222, 224: data communication
- 240: processor subsystem
- 260: communication interface
- 300: misaligned short axis cardiac MR cine slice stack
- 302: aligned short axis cardiac MR cine slice stack
- 310: part of cardiac structure
- 320: wall of cardiac structure
- 360, 362: surface of deformable surface model
- S1-S3: shift applied to image slice

- 400: set of adjacent image slices from a given cardiac phase
- 402: set of adjacent image slices from a further cardiac phase
- 410-412: image slices
- 420-424: sampling grid defining input to machine trained model

- 500: image slice before slice alignment
- 502: image slice after slice alignment
- 560: surface of deformable surface model

- 600: method for slice alignment using machine trained model
- 610: accessing image data of slice stack
- 620: accessing data of machine trained model
- 630: applying machine trained model to adjacent slices
- 640: shifting image slice(s)

- 700: method for slice alignment using surface model
- 710: accessing image data of slice stack
- 720: accessing data of deformable surface model
- 730: adapting the surface model to cardiac structure
- 740: shifting image slice(s)
- 750: repeated iterations

- 800: computer-readable medium
- 810: non-transitory data

### DETAILED DESCRIPTION OF EMBODIMENTS

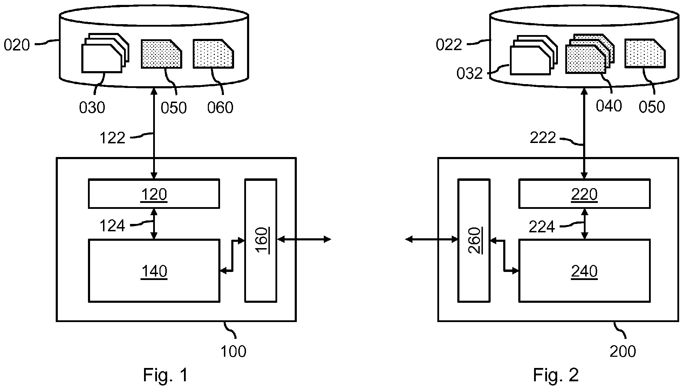

**Fig. 1** shows a system 100 for slice alignment of short axis cardiac magnetic resonance cine slice stacks, which in one embodiment may use a machine trained model and, in another embodiment, a deformable surface model to perform the slice alignment. The system 100 is shown to comprise an input interface 120 for accessing image data 030 of an input set of image slices acquired using a short axis cardiac magnetic resonance cine protocol. For example, as also shown in Fig. 1, the input interface 120 may provide data access 122 to an external data storage 020 which may comprise said image data 030. Alternatively, the input interface 120 may provide data access to an internal data storage which is part of the system 100. Alternatively, the image data 030 may be accessed via a network. In general, the input interface 120 may take various forms, such as a network interface to a local or wide area network, e.g., the Internet, a storage interface to an internal or external data storage, etc. The data storage 020 may take any known and suitable form.

The data storage is further shown to comprise trained model data 050 and surface model data 060 which are further explained in the following. Depending on the embodiment, the data storage may comprise one or both types of data 050, 060. In some embodiments, the image data 030, the trained model data 050 and the surface model data 060 may each be accessed from a different data storage.

The system 100 is further shown to comprise a processor subsystem 140 which may internally communicate with the input interface 120 via data communication 124, and as an optional component, a communication interface 160. As also shown in Fig. 1, the communication interface 160 may be an external communication interface such as a network interface to a local or wide area network, e.g., the Internet. In some embodiments, the input interface 120 may be the same interface as the communication interface 160. In other embodiments, the communication interface 160 may be a network interface via which data is received and the input interface 120 may be a data storage interface via which data is stored.

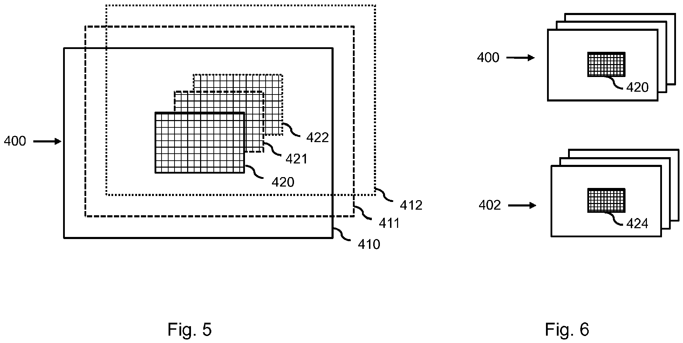

In one embodiment, the processor subsystem 140 may be configured to access the trained model data 050 which defines a machine trained model which is trained on training data comprising image data of a training set of image slices acquired using a short axis cardiac magnetic resonance cine protocol. Such a machine trained model may for example be obtained from the system described with reference to Fig. 2. The processor subsystem 140 may be configured to apply the machine trained model to sets of adjacent image slices of the input set of image slices, thereby obtaining at least one shift value for at least one of the image slices of the sets of adjacent image slices, and to shift said image slice based on the shift value. Various details and aspects of this embodiment, including optional aspects thereof, will be further elucidated with reference to, inter alia, Figs. 5 and 6.

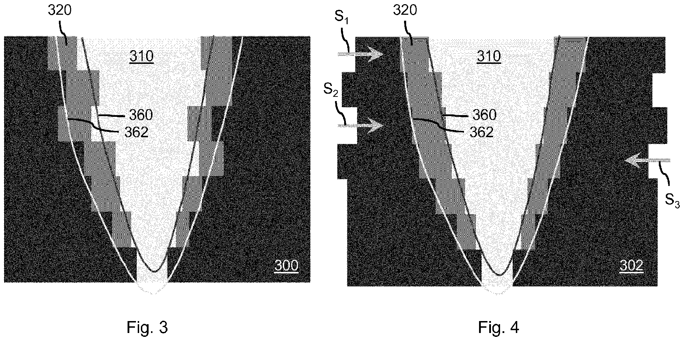

In another embodiment, the processor subsystem 140 may be configured to access the surface model data 060 defining a **deformable surface model** for segmenting a cardiac structure in short axis cardiac MR cine slice stacks. The deformability of the surface model may be constrained by shape regularization. The processor subsystem 140 may be configured to adapt the surface model to the cardiac structure by detecting boundary points of the cardiac structure in the image data and deforming the surface model towards the boundary points to obtain an adapted surface model which is adapted in shape to the cardiac structure in the image data, and to shift at least one image slice relative to other image slices so that the boundary points in the image slice obtain an improved match with a cross-sectional representation of the surface model in the respective image slice. Various details and aspects of this embodiment, including optional aspects thereof, will be further elucidated in this specification with reference to Figs. 3 and 4.

In general, the system 100 may be embodied as, or in, a single device or apparatus, such as a workstation, e.g., laptop or desktop-based, or a server. The device or apparatus may comprise one or more microprocessors which execute appropriate software. For example, the processor subsystem may be embodied by a single Central Processing Unit (CPU), but also by a combination or system of such CPUs and/or other types of processing units. The software may have been downloaded and/or stored in a corresponding memory, e.g., a volatile memory such as RAM or a non-volatile memory such as Flash. Alternatively, the functional units of the system, e.g., the input interface and the processor subsystem, may be implemented in the device or apparatus in the form of programmable logic, e.g., as a Field-Programmable Gate Array (FPGA). In general, each functional unit of the system may be implemented in the form of a circuit. It is noted that the system 100 may also be implemented in a distributed manner, e.g., involving different devices or apparatuses, such as distributed servers, e.g., in the form of cloud computing.

**Fig. 2** shows a system 200 for training a machine trainable model for slice alignment of short axis cardiac magnetic resonance cine slice stacks. The system 200 is shown to comprise an input interface 220 and a processor subsystem 240 which communicate via data communication 224. The system 200 is further shown to comprise a communication interface 260. The input interface 220 is shown to provide access to a data storage 022 via data communication 222. The thus-far described components of the system 200 may correspond in general type to the respective components of the system 100 of Fig. 1. In particular, the embodiment options described in the previous paragraph may also apply to the system 200.

However, unlike the system 100 of Fig. 1, the processor subsystem 240 of the system 200 is configured to train the machine trainable model, namely using training data which comprises image data 032 of a training set of image slices acquired using a short axis cardiac magnetic resonance cine protocol and in which one or more adjacent image slices are mutually misaligned. In addition, the training data used for the training may comprise shift values 040 for reducing said mutual misalignment by shifting one or more of the image slices. The processor subsystem 240 may be configured to train the machine trainable model using the training data to obtain a machine trained model, and in particular to obtain trained model data 050 representing the machine trained model. The machine trainable model may be of any suitable type, such as a neural network, for example a deep neural network (DNN) comprising one or more convolutional layers (Convolutional DNN). Such a neural network may be trained using any suitable machine learning technique.

**Fig. 3** shows a cross-section of a misaligned slice stack 300 which may be obtained by acquisition using a short axis cardiac magnetic resonance cine protocol. It can be seen that in such slice stacks, the boundary of a (part of a) cardiac structure 310 may be misaligned in a 'zig-zag' pattern between the individual slices, which is visible in Fig. 3 by the wall 320 of the cardiac structure 310, being in this example the myocardial wall, following this 'zig-zag' pattern. Such misalignment may hinder the adaptation of a deformable surface model to the image data, and thereby hinder the segmentation of the cardiac structure 310. Namely, such a deformable surface model may, when adapted to the image data, follow the general boundary of the cardiac structure 310 but may be prevented from exactly following the irregular 'zig-zag' misalignment pattern due to shape regularization. Fig. 3 indeed illustrates that surfaces 360, 362 of the deformable surface model may follow the general boundary of the cardiac structure 310 but may in individual slices often be misaligned with the wall 320 due to the aforementioned 'zig-zag' misalignment pattern. At the same time, the 'zig-zag' pattern may obfuscate finer anatomical details of the cardiac structure 310, and thereby prevent or hinder the further adaptation of the deformable surface model to such finer anatomical details.

The system 100 described with reference to Fig. 1 may in one embodiment perform slice alignment by adapting a deformable surface model to the boundary of the cardiac structure, e.g., as illustrated in Fig. 3, and then shift individual slices of the slice stack so that the boundary points contained in a respective slice better match the cross-section of the thus-far adapted deformable surface model in the slice, e.g., as illustrated in Fig. 4 by the arrows labelled S₁-S₃ denoting the direction in which a respective slice is shifted. It is noted that while Figs. 3 and 4 do not explicitly show such boundary points, these may be detected in a manner as known per se and may indicate to the system 100 where the boundary of the cardiac structure in each slice lies, being here the wall 320. Effectively, in this step of the slice alignment, individual slices may be adapted to the deformable surface model (by appropriate shifting) instead of the other way around. The overall slice alignment process may be performed iteratively, in that the deformable surface model may be adapted to the slice stack, individual slices may then be adapted to the deformable surface model (by appropriate shifting), after which the deformable surface model may be again adapted to the slice stack, etc. This process may be repeated, e.g., a fixed-number of times or until a convergence criterion is reached.

With continued reference to Figs. 3 and 4, it is noted that the applying of a deformable surface model to the image data for segmentation purposes is also known as model-based image segmentation. Model-based image segmentation typically uses a triangulated surface mesh, and may apply the surface mesh to the image data by iteratively detecting the boundary points in the image, for example per triangle and by searching for boundaries along the triangle normal, and subsequently adapting the surface mesh to the detected boundaries. A shape model may regularize the mesh deformations. The surface model-based slice alignment may be based on the observation that a surface model may roughly interpolate through the original slice stack. Due to the shape model regularization, an exact fit to the 'zig-zag' boundary of the cardiac structure may not be achieved. The approach shown in Fig. 3 and 4 may add a new step to this iterative adaptation. Namely, after the surface mesh has achieved an intermediate fit to the slice stack, the adaptation strategy may be changed: rather than attracting the triangles of the surface mesh to the detected boundary points, an individual slice may be shifted with its boundary points towards the surface mesh. To improve the overall results, mesh adaptation and slice shifts may be alternated in a configurable scheme. For example, after a start condition is satisfied, every second iteration may be used to shift the slices, while every other iteration may be used for further mesh adaptation. This may improve the slice alignment but also the segmentation of the cardiac structure, as better aligned slices may lead to improved boundary detection (due to interpolation between slices during boundary detection) and thereby to better mesh adaptation.

In a specific embodiment, the boundary detection for a triangle may be limited to searching for boundary points in the same slice, e.g., to avoid shifting slices in a plane that is far 'above or below' the corresponding triangle. The difference vector pointing from the triangle to the boundary may also be required to be reasonably parallel to the slice, e.g., as quantified by a corresponding metric, rather than pointing mostly along the stacking direction of the slices. This may improve the numerical stability of the slice alignment. Also, the boundary detection may be configured to reject doubtful boundaries, so that outliers may be filtered out.

As also referenced earlier, **Fig. 4** shows the slice stack of Fig. 3 after shifting several slices into alignment with the applied deformable surface model.

It is noted that even though the adapted surface model may generally follow the cardiac structure in the non-aligned slice stack and the estimated slice shifts are expected to roughly sum up to zero and thereby not introduce any tilting of the slice stack, actual slice shift estimates may be imperfect. Estimating and applying slice shifts over many iterations may therefore lead to a slow translational drift and/or tilting (skewing) of the slice stack, and the surface model may follow this drift when iteratively adapting the surface model to the slice stack. To compensate for such drift and/or tilting, an additional slice shift normalization step may be introduced which may comprise the following (described with reference to the x-component of the shift; the y-component may be normalized correspondingly, with *x* and y referring to the in-slice coordinate system): Let{*dx*[*z*]} be the estimated x-displacements for all slices indexed by *z.* Linearly transformed 'normalized' displacements may be defined as {*T*(*dx*[*z*],*z*)} with *T*(*dx*[*z*],*z*) = *dx*[*z*] + *a* · *z* + *b* where the linear transformation parameters *a* and b may be estimated such that ∑*_{Z}* ∑*_{Z}* T(dx[z],z) is minimized, e.g., such that *T*(*dx*[*z*],*z*) deviates as little as possible from a straight line along the slice normal (in z direction) centred at *x* = 0. This also implies ∑*_{Z}*T(dx[z],z) = 0, thereby eliminating translational drift. Such normalization may thereby remove an offset or a linear trend from a sequence of shift values which represents the shift values of a sequence of image slices.

With further reference to the slice alignment using a machine trained model, such as a Convolutional DNN, this approach considers slice shift estimation as a regression task that is solved using machine learning techniques. Here, pairs or n-tuples of successive slices may be input into a machine trained model that infers a relative shift that will bring the slices into an anatomically aligned position based on the image intensity values of the slices, and in some embodiments, based on auxiliary information. To train such a model, a slice stack aligned by the approach described with reference to Figs. 3 and 4 may be used as reference. For example, such a slice stack may be purposefully misaligned using known shift values, and the purposefully misaligned slice stack may together with the known shift values be used as training data for the machine trainable model to learn to predict the slice shifts.

**Fig. 5** shows a set of adjacent slices 400 and sampling grids 420-422 indicating which intensity values from individual image slices 410-412 are used as input to the machine trained model. For example, the sampling grid may be a pre-defined grid, e.g., using 256 x 256 sampling points at known intervals such as 1mm apart. For example, when intensity values of three adjacent slices are input to the machine trained model, the model may be fed with 3 x 256 x 256 intensity values. Various other sampling grids may be defined as well, e.g., involving a different number of samples, a different spatial shape, a different number of slices, etc. In some embodiments, differences in intensity values between the slices may be used as input. Intensity values may be normalized, e.g., using a statistic from the complete stack, e.g., mean and standard deviation or percentiles of intensities.

For the example of pair-wise estimation of slice shifts, the following is noted. For N slices, a global shift may be applied to all slices without changing the relative alignment or mis-alignment. However, the relative positions between the N slices may be defined by N-1 relative shifts. Such relative shifts may be expressed in various ways, e.g., as shift of each slice with respect to a reference slide (e.g., the first or last slice in the slice stack), or as the relative shift between successive slices i and i+1. Overall, one may thereby estimate N-1 relative slice shifts for the N slices. For one of the slices (e.g., the first or last slice), a zero shift may be assigned. However, this may lead to an overall shift of the estimated relative shifts for the other N-1 slices as these may not sum up to zero. Moreover, as also described earlier, slice shift estimates may be imperfect and introduce drift and/or tilting. The earlier-described slice shift normalization step may therefore be applied to the series of shift values obtained by the slice shift estimation to remove bias, e.g., an offset (the aforementioned global shift) or linear trend, from the shift values to minimize the deviation from the z-axis (referring to the axis orthogonal to the in-slice axes).

In addition to image intensities, various other types of auxiliary information may be used as well as input to the machine trained/trainable model. For example, the anatomical content of the slices may vary from the apex to the "base" (transition from ventricles to atria) and thereby vary quite significantly throughout the slice stack. The machine trained/trainable model may benefit from knowing that 'more apical' or 'more basal' slices have to be aligned. In addition, since the slices may show the heart in varying rotations, angular information may help as well.

The following refers to the machine trained/trainable model as a neural network, but also applies to other types of machine trained/trainable models.

There are various ways to provide such additional positional information to the neural network. In one embodiment, positional information may be provided to the neural network by indicating that a particular slice is at a particular relative position in the slice stack, e.g., by specifying a percentage with 0% being most apical or even below the apex and 100% being most basal or above the ventricles. In another embodiment, a 3D coordinate may be specified per sample point. For example, the DICOM information of the acquisition may be used so that a relative position may be specified with respect to the volume center in so-called 'patient' (or 'world') coordinates, where 'z' is from foot to head, 'x' is from right to left, and 'y' is from front to back of the patient. Since the heart has a typical orientation within each patient, such DICOM-based positional information may already provide a coarse indication of the anatomical content of a particular slice/sample point. However, both the size and the precise orientation of the heart varies from patient to patient, so that a more specific coordinate system may help. In a more elaborate embodiment, an approximate segmentation of the heart may be used (e.g., with imperfect or absent slice alignment) to define a coordinate system based on cardiac landmarks, e.g., as also used to define 2-, 3- and 4-chamber plus short axis views. Since this may require a complete heart segmentation, an intermediate embodiment may use a coordinate system estimated by a Generalized Hough Transform (GHT) that may be applied with various scales and various rotations of its edge template. The anatomical positional information of a slice or sample point may then be determined in this coordinate system and provided to the neural network as input.

Various types of pre-processing may be used as well before training or subsequently using the machine trained model. This may reduce the complexity of the learning problem and incorporate prior knowledge already in the input to the machine trained model. For example, the image data of the slice stack may be resampled in a new (anatomically aligned) coordinate system such that the cardiac anatomy follows a standard orientation in this coordinate system. Rather than sampling the image intensities on a grid aligned with the scan axes, the sampling grid may also be re-oriented in each individual slice using axes associated with anatomical directions. The only additional positional information input to the neural network may be the z-coordinate encoding the anatomical position of the slice.

Additionally or alternatively, a rough pre-segmentation or localization may be used to mask out irrelevant parts of the image data, such as the rib cage, which may exhibit a different transformation compared to the moving heart.

Knowledge of the orientation of the anatomy may also be used in recurrent architectures which predict a sequence of translations when the input consists of a sequence of successive (or even single) slices, for example always starting close to the apex and finishing around the basal parts of the heart. Even if there is no correlation between the translations from slice to slice, this may still exploit locational information along the heart's long axis. Namely, an internal state may be updated at each new slice, but information of the previous slices may be retained. Accordingly, the neural network may be aware of the current slice position which may make the output part of the neural network act differently. In this way, typical alignment curves across the population may be implicitly taken into account. Such an approach may also make use of so-called 'bi-currence', in which upward (from the apex) and downward (from the basal part) passes may be combined.

**Fig. 6** shows two sets of adjacent image slices 400, 402 which are from two slice stacks which are each acquired during different cardiac phases, and in which sampling grids 420, 424 are defined in both sets of adjacent image slices. Namely, image intensities from different cardiac phases may be used as joint input to the neural network. In the example of Fig. 6, intensity values from two cardiac phases (e.g., ED and ES) may be sampled on the same grid in each cardiac phase, e.g., using sampling grids 420 and 424. As such, each sample point used as input to the neural network may provide intensity values from both cardiac phases.

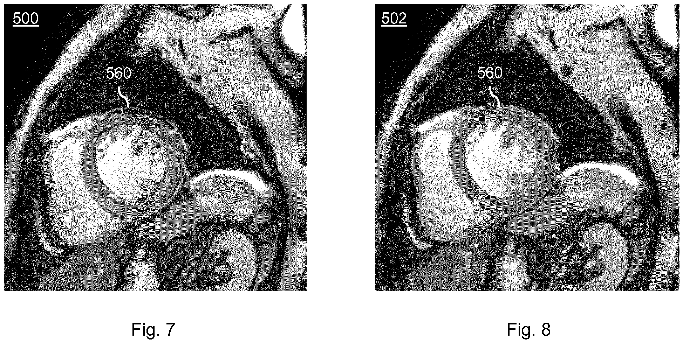

**Fig. 7** shows an image slice 500 of a cardiac structure and a cross-section of an adapted deformable surface model, and in particular of a surface 560 of the deformable surface model which is shown to misaligned with respect to the boundary of the cardiac structure due to a misalignment between image slices. **Fig. 8** shows a result of the slice alignment as described in this specification, in that the image slice of Fig. 7 is shown after shifting the image slice within the slice stack while the deformable surface model is shown after another iteration of adapting the deformable surface model to the slice stack. This may yield an improved alignment of the surface 500 to the boundary of the cardiac structure in the image slice 502.

In general, the slice alignment as described in this specification may be used in various medical systems and apparatuses, including but not limited to 3D visualization systems, e.g., 'orthoviewer' visualization systems which show two and four-chamber multiplanar reformats in addition to the individual slices.

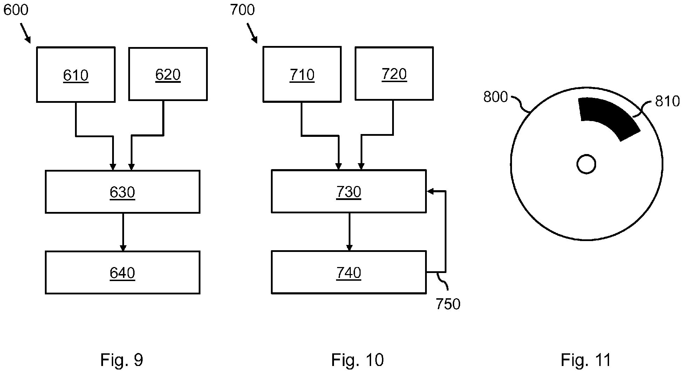

**Fig. 9** shows a flow-diagram of a computer-implemented method 600 for slice alignment using a machine trained model. The method 600 may correspond to an operation of the system 100 of Fig. 1 when configured for slice alignment using a machine trained model. However, this is not a limitation, in that the method 600 may also be performed using another system, apparatus or device.

The method 600 may comprise, in an operation titled "ACCESSING IMAGE DATA OF SLICE STACK", accessing 610 image data of an input set of image slices acquired using a short axis cardiac magnetic resonance cine protocol. The method 600 may further comprise, in an operation titled "ACCESSING DATA OF MACHINE TRAINED MODEL", accessing 620 trained model data defining a machine trained model, wherein the machine trained model is trained on training data comprising image data of a training set of image slices acquired using a short axis cardiac magnetic resonance cine protocol, wherein one or more adjacent image slices are mutually misaligned, and wherein the training data further comprises shift values for reducing said mutual misalignment by shifting one or more of the image slices. The method 600 may further comprise, in an operation titled "APPLYING MACHINE TRAINED MODEL TO ADJACENT SLICES", applying 630 the machine trained model to sets of adjacent image slices of the input set of image slices, thereby obtaining at least one shift value for at least one of the image slices of the sets of adjacent image slices, and in an operation titled "SHIFTING IMAGE SLICE(S)", shifting 640 said image slice based on the shift value.

**Fig. 10** shows a flow-diagram of a computer-implemented method 700 for slice alignment using a machine trained model. The method 700 may correspond to an operation of the system 100 of Fig. 1 when configured for slice alignment using a deformable surface model. However, this is not a limitation, in that the method 700 may also be performed using another system, apparatus or device.

The method 700 may comprise, in an operation titled "ACCESSING IMAGE DATA OF SLICE STACK", accessing 710 image data representing a set of image slices acquired using a short axis cardiac magnetic resonance cine protocol. The method 700 may further comprise, in an operation titled "ACCESSING DATA OF DEFORMABLE SURFACE MODEL", accessing 720 surface model data defining a deformable surface model for segmenting a cardiac structure in short axis cardiac MR cine slice stacks, wherein deformability of the surface model is constrained by shape regularization. The method 700 may further comprise, in an operation titled "ADAPTING THE SURFACE MODEL TO CARDIAC STRUCTURE", adapting 730 the surface model to the cardiac structure by detecting boundary points of the cardiac structure in the image data and deforming the surface model towards the boundary points to obtain an adapted surface model which is adapted in shape to the cardiac structure in the image data. The method 700 may further comprise, in an operation titled "SHIFTING IMAGE SLICE(S)", shifting 740 at least one image slice relative to other image slices so that the boundary points in the image slice obtain an improved match with a cross-sectional representation of the surface model in the respective image slice. Operations 730, 740 may be repeated in iterations 750.

It will be appreciated that, in general, the operations of method 600 of Fig. 9 and/or method 700 of Fig. 10 may be performed in any suitable order, e.g., consecutively, simultaneously, or a combination thereof, subject to, where applicable, a particular order being necessitated, e.g., by input/output relations.

The method(s) may be implemented on a computer as a computer implemented method, as dedicated hardware, or as a combination of both. As also illustrated in **Fig. 11****,** instructions for the computer, e.g., executable code, may be stored on a computer readable medium 800, e.g., in the form of a series 810 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 11 shows an optical disc 800. Alternatively, the computer readable medium 800 may comprise transitory or non-transitory data 810 representing the machine trained model as described elsewhere in this specification.

Examples, embodiments or optional features, whether indicated as nonlimiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of' when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for slice alignment of short axis cardiac magnetic resonance cine slice stacks, comprising:
- an input interface (120) for accessing image data (030) of an input set of image slices acquired using a short axis cardiac magnetic resonance cine protocol;
- a processor subsystem (140) configured to:
- access trained model data (050) defining a machine trained model, wherein the machine trained model is trained on training data comprising image data of a training set of image slices acquired using a short axis cardiac magnetic resonance cine protocol, wherein one or more adjacent image slices are mutually misaligned, and wherein the training data further comprises shift values for reducing said mutual misalignment by shifting one or more of the image slices;
- apply the machine trained model to sets of adjacent image slices (400) of the input set of image slices, thereby obtaining at least one shift value for at least one of the image slices of the sets of adjacent image slices; and
- shift said image slice based on the shift value.

2. The system (100) according to claim 1, wherein the processor subsystem (140) is configured to:
- apply the machine trained model to the sets of adjacent image slices (400) of the input set of image slices to obtain a series of shift values;
- remove an offset or linear trend from the series of shift values;
- shift respective image slices of the sets of adjacent image slices based on respective shift values of the series of shift values.

3. The system (100) according to claim 1 or 2, wherein the machine trained model is configured and trained to use as further input positional information which is indicative of a position of respective sets of adjacent image slices relative to a cardiac structure which is shown in the input set of image slices.

4. The system (100) according to any one of claims 1 to 3, wherein the machine trained model is configured and trained to use as further input angular information which is indicative of an orientation of a cardiac structure which is shown in the input set of image slices, relative to a coordinate system associated with the input set of image slices.

5. The system (100) according to claim 3 or 4, wherein the processor subsystem (140) is configured to obtain at least one of the positional information and the angular information by segmenting the cardiac structure in the input set of image slices, for example by applying a deformable surface model to the image data.

6. The system (100) according to claim 5, wherein the processor subsystem (140) is configured to mask a part of the image data of the input set of image slices which does not belong to the cardiac structure before applying the machine trained model to the sets of adjacent image slices of the input set of image slices.

7. The system (100) according to any one of claims 1 to 6, wherein the input set of image slices is a first set of image slices (400), wherein the input interface is configured for accessing image data of a second set of image slices (402) acquired during a different cardiac phase than the first set of image slices, and wherein the machine trained model is configured and trained to use spatially corresponding samples of the first set of image slices and the second set of image slices as joint input.

8. A computer-readable medium (800) comprising transitory or non-transitory data (810) defining a machine trained model, wherein the machine trained model is configured and trained to be applied to a set of adjacent image slices of a set of image slices acquired using a short axis cardiac magnetic resonance cine protocol, wherein the machine trained model is trained to output a shift value if the set of adjacent image slices is mutually misaligned for reducing mutual misalignment.

9. A system (100) for slice alignment of short axis cardiac magnetic resonance cine slice stacks, comprising:
- an input interface (120) for accessing image data (030) representing a set of image slices acquired using a short axis cardiac magnetic resonance cine protocol;
- a processor subsystem (140) configured to:
- access surface model data (060) defining a deformable surface model for segmenting a cardiac structure in short axis cardiac MR cine slice stacks, wherein deformability of the surface model is constrained by shape regularization;
- adapt the surface model to the cardiac structure by detecting boundary points of the cardiac structure in the image data and deforming the surface model towards the boundary points to obtain an adapted surface model which is adapted in shape to the cardiac structure in the image data; and
- shift (S1-S3) at least one image slice relative to other image slices so that the boundary points in the image slice obtain an improved match with a cross-sectional representation of the surface model in the respective image slice.

10. The system (100) according to claim 9, wherein the processor subsystem (140) is configured to deform the surface model towards the boundary points of the cardiac structure based on a cost function penalizing a distance of the surface model to the boundary points, and to shift the at least one image slice relative to the other image slices so that the match is improved according to the cost function.

11. The system (100) according to claim 9 or 10, wherein the processor subsystem (140) is configured for iterative slice alignment by repeating said adapting of the surface model and said shifting of the at least one image slice at least twice.

12. The system (100) according to any one of claims 9 to 11, wherein the processor subsystem (140) is configured to:
- after adapting the surface model, obtain a series of shift values for respective image slices of the set of image slices to obtain the improved match with the sectional representation of the surface model in the respective image slices;
- remove an offset or linear trend from the series of shift values;
- shift the respective image slices based on the respective shift values of the series of shift values.

13. A computer-implemented method (600) for slice alignment of short axis cardiac magnetic resonance cine slice stacks, comprising:
- accessing (610) image data of an input set of image slices acquired using a short axis cardiac magnetic resonance cine protocol;
- accessing (620) trained model data defining a machine trained model, wherein the machine trained model is trained on training data comprising image data of a training set of image slices acquired using a short axis cardiac magnetic resonance cine protocol, wherein one or more adjacent image slices are mutually misaligned, and wherein the training data further comprises shift values for reducing said mutual misalignment by shifting one or more of the image slices;
- applying (630) the machine trained model to sets of adjacent image slices of the input set of image slices, thereby obtaining at least one shift value for at least one of the image slices of the sets of adjacent image slices; and
- shifting (640) said image slice based on the shift value.

14. A computer-implemented method (700) for slice alignment of short axis cardiac magnetic resonance cine slice stacks, comprising:
- accessing (710) image data representing a set of image slices acquired using a short axis cardiac magnetic resonance cine protocol;
- accessing (720) surface model data defining a deformable surface model for segmenting a cardiac structure in short axis cardiac MR cine slice stacks, wherein deformability of the surface model is constrained by shape regularization;
- adapting (730) the surface model to the cardiac structure by detecting boundary points of the cardiac structure in the image data and deforming the surface model towards the boundary points to obtain an adapted surface model which is adapted in shape to the cardiac structure in the image data; and
- shifting (740) at least one image slice relative to other image slices so that the boundary points in the image slice obtain an improved match with a cross-sectional representation of the surface model in the respective image slice.

15. A computer-readable medium (800) comprising transitory or non-transitory data (810) representing a computer program, the computer program comprising instructions for causing a processor system to perform the method according to claim 13 or 14.
